# EUROPÄISCHE PATENTSCHRIFT

(11) **EP 4 072 648 B1**
(45) Veröffentlichungstag und Bekanntmachung des Hinweises auf die Patenterteilung: **28.01.2026**
(21) Anmeldenummer: 20838903.1
(22) Anmeldetag: 10.12.2020
(51) Int. Cl.: A61M 60/104, A61M 60/279, A61M 60/835, A61M 60/845

(54) **SCHLAUCHPUMPE ZUM FÖRDERN VON MEDIZINISCHEN FLUIDEN**
PERISTALTIC PUMP FOR CONVEYING MEDICAL FLUIDS
POMPE PÉRISTALTIQUE POUR CONVOYER DES FLUIDES MÉDICAUX

(30) Priorität: 11.12.2019 DE 102019133969
(43) Veröffentlichungstag der Anmeldung: 19.10.2022
(73) Patentinhaber: Fresenius Medical Care Deutschland GmbH, 61352 Bad Homburg (DE)
(72) Erfinder: SPENGLER, Gerhard, 97502 Euerbach (DE); KIRSTGEN, Udo, 72108 Rottenburg (DE)
(74) Vertreter: Bobbert & Partner Patentanwälte PartmbB
(86) Internationale Anmeldenummer: PCT/EP2020/085589
(87) Internationale Veröffentlichungsnummer: WO 2021/116316

(56) Entgegenhaltungen:
- EP-A1- 2 514 451

## Beschreibung

Die vorliegende Erfindung betrifft eine medizinische Fördervorrichtung zum Fördern eines in einem Schlauch geführten Fluids gemäß Anspruch 1. Sie betrifft zudem eine Blutbehandlungsvorrichtung gemäß Anspruch 14 bzw. jeweils den Oberbegriffen der vorgenannten Ansprüche.

Aus der medizintechnischen Praxis sind Schlauchpumpen bzw. Peristaltikpumpen zur Förderung eines in einem Schlauch geführten Fluids bekannt. Schlauchpumpen sind eine spezielle Form der Verdrängerpumpen, bei denen der Arbeitsraum aus dem Lumen eines flexiblen Schlauchs besteht. Die Veränderung des Arbeitsraumes erfolgt über eine von außen bedingte mechanische Verformung mittels Okklusionsvorrichtungen (beispielsweise in Form von Okklusionsrollen oder -flächen). Zur Erzeugung eines Volumenstroms wird der Schlauch an einer Stelle oder mehreren Stellen (Okklusionsbereich) okkludiert, und dieser Okklusionsbereich wird in Richtung Pumpenausgang entlang des Schlauchs vorwärts bewegt. Das Ansaugen von neuem Fluid am Pumpeneingang erfolgt durch Unterdruck, u. a. bedingt durch die Rückverformung des nun nicht mehr okkludierten Schlauchbereichs infolge der Elastizität des Schlauchs.

Aus der EP 2 514 451 A1 ist ein Pumpschlagsegment für einen Blutschlauchsatz bekannt.

Eine Aufgabe der vorliegenden Erfindung ist es, eine weitere Fördervorrichtung vorzuschlagen. Zudem soll eine Blutbehandlungsvorrichtung angegeben werden.

Die erfindungsgemäße Aufgabe wird durch eine Fördervorrichtung mit den Merkmalen des Anspruchs 1 gelöst. Sie wird ferner gelöst durch eine Blutbehandlungsvorrichtung mit den Merkmalen des Anspruchs 14.

Die erfindungsgemäße Fördervorrichtung dient zum Fördern eines in einem Schlauch geführten medizinischen Fluids. Das Fluid kann beispielsweise Vollblut, Plasma oder sonstige Blutbestandteile, Pharmazeutika, Substituat, Dialysat, parenterale Nahrungsmittel, oder ein sonstiges medizinisches Fluid sein oder umfassen.

Die erfindungsgemäße Fördervorrichtung umfasst einen Rotor mit Okklusionsvorrichtungen und einen Stator. Der Stator kann beispielsweise das Pumpengehäuse oder ein Teil hiervon sein. Er bildet oder umfasst eine Aufnahme für den Rotor und den Schlauch. So umfasst der Stator mindestens einen Statorboden, ein Schlauchbett und einen Raum für die Aufnahme des Schlauchs, wobei das Schlauchbett ein Gegenlager für die Okklusionsvorrichtungen des Rotors ist oder umfasst. Der Stator kann, z. B. in einer Richtung der Drehachse des Rotors, zugänglich oder offen sein, oder ein schließbares und/oder abnehmbares Abdeckelement aufweisen, welche den Rotor und den Schlauch u. a. vor einer versehentlichen Berührung schützt. Der Stator umfasst ferner einen Eintritts- und einen Austrittsbereich für den Schlauch, welche beispielsweise als Pumpeneingang und Pumpenausgang ausgestaltet sein können. Die Fördervorrichtung saugt medizinische Fluide aus dem Eintrittsbereich an und fördert sie weiter zum Austrittsbereich.

Der Rotor umfasst wenigstens eine Rotorachse (welche beispielsweise als geometrische Achse einer Nabenbohrung ausgestaltet sein kann, oder statt einer Achse hierin alternativ eine Rotorwelle sein kann), welche insbesondere zentral (etwa bezogen auf den Rotor) angeordnet ist, und wenigstens zwei Okklusionsvorrichtungen, beispielsweise in Form von Rollen. Die Okklusionsvorrichtungen, welche radial zur Rotorachse angebracht sind, dienen im Gebrauch der Fördervorrichtung dazu, den Schlauch intermittierend gegen das Gegenlager des Stators zu komprimieren und die Fluide im Schlauchlumen Richtung Pumpenausgang zu drücken oder zu fördern. Dabei entsteht stromaufwärts des Okklusionsbereichs ein Unterdruck im Schlauch, der weiteres zu förderndes Fluid ansaugt.

Der Schlauch wird alternierend komprimiert und darf wieder dekomprimieren. Der Rotor ist nicht über seinen gesamten Umfang mit Okklusionsvorrichtungen bestückt, sondern diese sind mit Zwischenräumen angeordnet, die bei der Rotation die intermittierende Okklusion des Schlauchs ermöglichen. Derartige Okklusionsvorrichtungen können in unterschiedlicher Anzahl vorhanden sein. Sie können beispielsweise um 180° zueinander versetzt sein bei Rotoren mit zwei Okklusionsvorrichtungen, beispielsweise um 120° bei Rotoren mit drei Okklusionsvorrichtungen, beispielsweise um 90° bei Rotoren mit vier Okklusionsvorrichtungen, usw. Der Abstand zwischen den Okklusionsvorrichtungen ist in einigen Ausführungsformen gleich, in anderen jedoch nicht.

Der Rotor weist erfindungsgemäß ferner wenigstens ein Führungselement auf, welches um seine Längsachse drehbar angeordnet ist und hierin als axiales Führungselement für den Schlauch bezeichnet wird. Es dient dazu, den Schlauch im Schlauchbett des Stators auszurichten und/oder zu zentrieren.

Die Längsachse des axialen Führungselements verläuft vorzugsweise parallel zur Längsachse der Rotorachse.

Da sich das axiale Führungselement erfindungsgemäß um seine Längsachse drehen kann, dient es vorzugsweise dazu, auf den Schlauch wirkende, durch die Rotationsbewegung des Rotors erzeugte Reibungskräfte zu reduzieren.

Die Drehbewegungen des axialen Führungselements können passiv sein, d. h. ohne eigenen Antrieb, also nur aufgrund der Reibungskräfte gegen den Schlauch entstehen.

In bestimmten Ausführungsformen können genauso viele axiale Führungselemente vorgesehen sein, wie der Rotor Okklusionsvorrichtungen aufweist. Damit kann, bei laufendem Rotor, jeder Okklusionsvorrichtung ein axiales Führungselement vorauseilen.

In weiteren Ausführungsformen entspricht die Anzahl der Okklusionsvorrichtungen nicht der Anzahl an axialen Führungselementen. So können beispielsweise zwei oder mehr axiale Führungselemente vor jeder Okklusionsvorrichtung vorgesehen sein.

Das axiale Führungselement umfasst hierzu wenigstens einen mittleren (alternativ: zentralen) Abschnitt. Dieser kann drehbar angeordnet oder gelagert sein. Das axiale Führungselement weist ferner wenigstens einen ersten, radial über den mittleren Abschnitt überstehenden Seitenabschnitt auf, der rotierbar angeordnet ist. Dabei kann der erste Seitenabschnitt radial über einen Abschnitt eines Außenrands des Rotors überstehen.

Der Seitenabschnitt und der mittlere Abschnitt des axialen Führungselements nehmen den Schlauch auf, indem sie ihn von unten (in einer Seitenansicht des Rotors bei aufrechtstehender Drehachse desselben) und an der radial inneren Seite des Schlauchs begrenzen.

Seitenabschnitte und mittlere Abschnitte können in einigen Ausführungsformen ein einteiliges oder einstückiges Bauteil sein. Sie können alternativ mehrteilig oder zu einer Einheit zusammengesetzt sein.

Die erfindungsgemäße Blutbehandlungsvorrichtung weist eine erfindungsgemäße Fördervorrichtung auf. Die Fördervorrichtung kann Bestandteil der Blutbehandlungsvorrichtung sein, oder kann als Kassette oder Modul ausgestaltet sein, welche(s) an der Blutbehandlungsvorrichtung angebracht wird oder ist, beispielsweise an oder in einer dafür vorgesehenen Aufnahme der Blutbehandlungsvorrichtung mit beispielsweise einem Getriebe, an dem der Rotor der Fördervorrichtung angeschlossen wird bzw. werden kann.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können eines oder mehrere der vorstehend oder im Folgenden genannten Merkmale aufweisen. Dabei können die hierin genannten Merkmale in beliebiger Kombination Gegenstand von erfindungsgemäßen Ausführungsformen sein, sofern der Fachmann eine konkrete Kombination nicht als technisch unmöglich erkennt. Die Erfindung wird in den angehängten Ansprüchen definiert.

Erfindungsgemäße Ausführungsformen sind ferner Gegenstand der Unteransprüche und Ausführungsformen.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin von einer Ausführungsform die Rede ist, so handelt es sich um eine beispielhafte, erfindungsgemäße Ausführungsform.

Die Angaben "oben" und "unten" sind hierin bei Zweifel des Fachmanns als absolute oder relative Raumangaben zu verstehen, welche sich auf die Ausrichtung des betreffenden Bauteils in den Figuren bzw. auf die Ausrichtung des betreffenden Bauteils während seines üblichen Gebrauchs beziehen.

Wenn hierin die Rede von einer Bohrung ist, so kann diese alternativ eine Öffnung oder Durchgangsöffnung sein.

In einigen Ausführungsformen weist die erfindungsgemäße Fördervorrichtung wenigstens ein weiteres axiales Führungselement auf. In manchen Ausführungsformen ist wenigstens eines der axialen Führungselemente oder wenigstens eine der Okklusionsvorrichtungen an einer optionalen Schwinge des Rotors angeordnet.

Die Schwinge ist in einigen Ausführungsformen als Aufhängung, z. B. als Gabel, ausgestaltet, mit einem Schwinggelenk ausgestaltet, welches die Aufhängung bzw. die Gabel mit einem weiteren Abschnitt des Rotors, z. B. einem Nabenkörper, verbindet.

Ein Verbindungselement kann vorgesehen sein, mittels welchem die Okklusionsvorrichtung an der Aufhängung befestigt ist, wobei die Okklusionsvorrichtung um dieses Verbindungselement frei rotierend angeordnet sein kann. Das Verbindungselement kann die Drehachse der Okklusionsvorrichtung bilden.

Der optionale Nabenkörper koppelt die Schwinge und damit alle an ihr angeordneten Teile an die Achse des Rotors an. Der Nabenkörper koppelt somit in einigen Ausführungsformen beispielsweise alle Komponenten des Rotors, die sich innerhalb des Rotors oder von diesem ausgehend radial erstrecken, insbesondere die Okklusionsvorrichtungen und die axialen Führungselemente, an die Rotation desselben an.

Der optionale Nabenkörper ist in bestimmten Ausführungsformen als Verbindungsplatte ausgestaltet, d. h. als Platte mit den erforderlichen Bohrungen, Stiften oder sonstigen Verbindungsstrukturen, um die Rotorachse mit der/den Okklusionsvorrichtung(en) derart zu verbinden, dass die Bewegung der Rotorachse auf die Okklusionsvorrichtung(en) übertragen wird.

Schwingen sind in einigen Ausführungsformen als Arme ausgestaltet. Sie können dann rigide Strukturen bilden.

In einigen Ausführungsformen ist wenigstens eine Schwinge federnd angeordnet. Eine geringe Federung der Schwinge(n) kann beispielsweise vorteilhaft dazu beitragen, das Einlegen eines Schlauchs in den Rotor zu erleichtern, oder den Rotor auf einfache Weise an unterschiedliche Schläuche anzupassen.

In manchen Ausführungsformen sind wenigstens zwei axiale Führungselemente und/oder wenigstens zwei Okklusionsvorrichtungen einander gegenüberliegend an einer gemeinsamen Schwinge des Rotors angeordnet.

Hierbei kann der Rotor eine Schwinge für die Okklusionsvorrichtung(en) und eine weitere Schwinge für das/die axialen Führungselement(e) aufweisen.

Sowohl die Okklusionsvorrichtungen als auch die axialen Führungselemente können in unterschiedlicher Anzahl vorhanden sein. Sie können in ihrer radialen Anordnung an ihrer jeweiligen Schwinge mit stets gleichem Abstand oder in unterschiedlichen Abständen zueinander angeordnet sein.

In einigen Ausführungsformen sind mehrere Okklusionsvorrichtungen und mehrere axiale Führungselemente des Rotors an einer gemeinsamen Schwinge angeordnet. Optional ist hierbei jeder Okklusionsvorrichtung jeweils ein axiales Führungselement zugeordnet.

Die Okklusionsvorrichtungen und die axialen Führungselemente können erfindungsgemäß in unterschiedlicher Zahl vorhanden sein, und sie können in gleichen oder in unterschiedlichen Abständen zueinander in der Schwinge angeordnet sein.

In weiteren Ausführungsformen ist die Anzahl der Okklusionsvorrichtungen und der axialen Führungselemente nicht gleich. So können beispielsweise zwei oder mehr axiale Führungselemente vor jeder Okklusionsvorrichtung im Rotor angeordnet sein.

Die Schwinge kann hier alle dem Stand der Technik bekannten Ausgestaltungen haben.

In manchen Ausführungsformen kann wenigstens eines der axialen Führungselemente wenigstens einen zweiten Seitenabschnitt aufweisen. Dieser Seitenabschnitt kann kegelförmig ausgestaltet sein. Die geometrische Ausgestaltung der axialen Führungselemente dient einer besseren Führung des Schlauchs über dem Statorboden und um den Rotor, um die Quergleitbewegungen des Schlauchs beim laufenden Rotor zu minimieren und damit auch den Abrieb des Schlauchs zu reduzieren.

In einer weiteren Ausführungsform umfasst das axiale Führungselement einen mittleren Abschnitt, der leicht sanduhrförmig ist.

In einigen Ausführungsformen können der erste und/oder der zweite Seitenabschnitt des axialen Führungselements kegelförmig sein. Sie können zusammen einen Doppelkegel bilden, wobei die Spitzen der Kegel zueinander gewandt sind und wobei der mittlere Abschnitt des axialen Führungselements zwischen dem ersten und dem zweiten Seitenabschnitt angeordnet ist und diese verbindet.

Das doppelkegelförmige axiale Führungselement führt aufgrund seiner geometrischen Ausgestaltung den Schlauch um den Rotor. Auf weitere Führungselemente, welche demselben Zweck dienen würden, kann in solchen erfindungsgemäßen Ausführungsformen vorteilhafterweise verzichtet werden.

In manchen Ausführungsformen sind die beiden kegelförmigen Seitenabschnitte unterschiedlich, oder voneinander oder zueinander verschieden, das heißt, dass der aus den beiden kegelförmigen Seitenabschnitten gebildete Doppelkegel vorzugsweise nicht punktsymmetrisch zu seinem geometrischen Schwerpunkt ist.

In bestimmten Ausführungsformen umfasst der Rotor axiale Führungselemente, welche zusätzlich zum mittleren Abschnitt nur genau einen Seitenabschnitt, nicht aber zwei Seitenabschnitte, umfassen.

In einigen Ausführungsformen kann der Rotor ferner wenigstens ein laterales Führungselement umfassen.

Eine Längsachse des lateralen Führungselements verläuft vorteilhafterweise senkrecht zur Rotorachse und ist vor oder über dem Statorboden des Stators angeordnet, so dass der Schlauch zwischen dem Statorboden und dem lateralen Führungselement angeordnet ist. Das laterale Führungselement grenzt damit den Schlauch nur nach außen hin ab, d. h. zum Benutzer hin. Es kann vorteilhaft dazu beitragen, zu verhindern, dass der Schlauch bei rotierendem Rotor aus dem Statorboden des Stators herausgleitet.

Zum Statorboden hin können in solchen Ausführungsformen die axialen Führungselemente zusätzlich zum mittleren Abschnitt nur genau einen Seitenabschnitt umfassen, welcher einen Kontakt des Schlauchs mit dem Statorboden verhindert. Der mittlere Abschnitt des axialen Führungselements stützt und führt den Schlauch von unten (d. h. er stützt jene Fläche des Schlauchs, die der Rotorachse am nächsten angeordnet ist). Das laterale Führungselement verhindert seinerseits den Kontakt mit dem Nabenkörper des Rotors und verhindert, dass der Schlauch über die Okklusionsrolle hinausläuft. So wird der Schlauch in solchen Ausführungsformen nach außen, also dem Benutzer zugewandt, vorzugsweise ausschließlich, durch eines oder mehrere solcher lateralen Führungselemente gestützt und geführt.

In manchen Ausführungsformen kann das laterale Führungselement um seine Mittelachse drehbar sein. Dies kann dazu dienen, beim laufenden Rotor die mechanische Belastung für den eingelegten Schlauch zu verringern oder zu minimieren.

In einigen Ausführungsformen ist optional nur der mittlere Abschnitt des axialen Führungselements um seine Mittellängsachse drehbar. Auch dies kann bereits dazu beitragen, die mechanische Belastung, die auf den Schlauch wirkt, zu verringern.

In manchen Ausführungsformen ist die erfindungsgemäße Fördervorrichtung mit einem Abdeckelement ausgestattet. Das Abdeckelement kann den Rotor und/oder den Schlauch, soweit er in die Fördervorrichtung eingelegt ist, abdecken oder zumindest teilweise bedecken.

In einigen Ausführungsformen ist das Abdeckelement angeordnet, um bei fördernder Fördervorrichtung relativ zum Stator still zu stehen und/oder nicht-rotierbar zu sein.

In weiteren Ausführungsformen ist das Abdeckelement am Rotor angekoppelt und wird von ihm bei dessen Rotation mitgenommen.

Das Abdeckelement kann dazu geeignet sein, den Rotor oder die gesamte Fördervorrichtung vor Schmutz oder Feuchtigkeit zu schützen. Ferner kann der Benutzer beispielsweise davor bewahrt werden, versehentlich bei rotierendem Rotor zwischen Komponenten zu greifen, von denen wenigstens eine rotiert.

In einigen Ausführungsformen weist das Abdeckelement optional wenigstens eine Führungslasche auf oder besteht aus einer solchen. Die Führungslasche kann durch ihre Geometrie einem einfachen und sicheren Einbringen des Schlauchs in den Statorboden dienen.

Die Führungslasche kann z. B. als eine Absenkung eines Abschnitts des Abdeckelements zum Statorboden hin ausgestaltet sein. Sie kann beim Einlegen des Schlauchs eine stützende Aufnahme für jenen Abschnitt des Schlauchs sein, der um den Rotor herum eingelegt werden soll.

Beim Einlegen des Schlauchs in die Fördervorrichtung wird der Schlauch in Kontakt mit der Führungslasche gelegt. Dann wird der Schlauch in Richtung auf den Statorboden hin vorgeschoben. Der Schlauch gleitet z. B. mittels einer abgerundeten, glatten und/oder in der Richtung des Statorbodens abfallenden Fläche der Führungslasche in die gewünschte Position relativ zum Rotor und kann anschließend weiter um den Rotor herum vorgeschoben werden.

Die Geometrie der Führungslasche erleichtert vorzugsweiseweise das Einlegen des Schlauchs und erschwert, dass sich der Schlauch verdreht und/oder während des Einlegens aus dem Raum zwischen Rotor und Stator wieder herausrutscht.

In weiteren Ausführungsformen ist die Führungslasche in das Abdeckelement integriert und/oder bildet mit diesem eine einstückige Einheit.

In einigen Ausführungsformen bilden die Führungslasche(n) und das Abdeckelement eine mehrstückige Einheit.

Die Führungslasche kann in manchen Ausführungsformen als Einlege- oder Einfädelhilfe verstanden werden.

In manchen Ausführungsformen sind optional wenigstens ein axiales Führungselement und/oder ein laterales Führungselement in das Abdeckelement integriert.

In solchen Ausführungsformen sind Schwingen oder ähnliche Verbindungselemente zwischen Rotor und/oder axialen und/oder lateralen Führungselementen nicht mehr nötig, da die Aufgabe dieser Verbindungselemente vom Abdeckelement mitübernommen wird.

In manchen Ausführungsformen ist das Abdeckelement als Nabenkörper ausgestaltet. Es kann ein Verbindungselement für die Verbindung mit dem Getriebe der Fördervorrichtung umfassen. Es kann auch dazu die Rolle der Schwinge übernehmen; beispielsweise kann das Abdeckelement mit Stiften versehen sein, welche als Achse für die drehbaren Bauteile der Fördervorrichtung dienen können. Somit kann bei der Konstruktion der Fördervorrichtung vorteilhaft auf Schwingen, Naben oder ähnliche Verbindungselemente verzichtet werden, da die Verbindungs- und Ankoppelungsfunktion, die üblicherweise die Schwinge übernehmen würde, hier vom Abdeckelement übernommen wird.

In manchen Ausführungsformen ist die Fördervorrichtung eine Kassette oder ein Teil hiervon, welche vorbereitet ist, um an einer Fluidbehandlungsvorrichtung angebracht zu werden.

In einigen Ausführungsformen ist die Fördervorrichtung fest mit weiteren Abschnitten der Fluidbehandlungsvorrichtung, etwa deren Gehäuse, verbunden.

In manchen Ausführungsformen ist die Fluidbehandlungsvorrichtung eine Blutbehandlungsvorrichtung.

In einigen Ausführungsformen ist die Blutbehandlungsvorrichtung ausgestaltet als Dialysevorrichtung, Hämodialysevorrichtung, Hämofiltrationsvorrichtung, Hämodiafiltrationsvorrichtung, Apheresevorrichtung, Plasmabehandlungs- oder Austauschvorrichtung, TPE-Vorrichtung (Totale parenterale Ernährung), und/oder als Kombinationen hiervon.

In manchen Ausführungsformen ist der Abstand zwischen den Okklusionsvorrichtungen und dem Schlauchbett als Gegenlager der Okklusionsvorrichtungen nicht variabel.

In einigen Ausführungsformen verfügt das Schlauchbett über keine konische oder kegelförmige Stellfläche, welche sich gegen eine komplementär geformte Stützfläche am Gehäuse abstützt.

In manchen Ausführungsformen ist der Abstand zwischen den Okklusionsvorrichtungen und dem Schlauchbett nicht durch Verschieben des Schlauchbettes gegenüber dem Gehäuse entlang der Stützfläche einstellbar.

Manche oder alle Ausführungsformen können einen, mehrere oder alle der im Folgenden genannten Vorteile aufweisen.

Die erfindungsgemäße Fördervorrichtung erlaubt es, als Schlauchpumpe, Fluide schonend und ohne Kontakt mit anderen Bestandteilen der Vorrichtung als dem Inneren des Schlauchs zu fördern.

Der Aufbau der erfindungsgemäßen Fördervorrichtung kann ferner den Schlauch vor einer starken Beanspruchung oder gar Schädigung bewahren, indem Scherkräfte, welche durch das Verschieben des okkludierten Abschnittes des Schlauchs entlang der Schlauchlängsachse auftreten, gering gehalten werden können. Scherbeanspruchungen werden nicht nur für eine verringerte Schlauchlebensdauer verantwortlich gemacht, sondern auch - bei zunehmendem Schlauchverschleiß - für eine Abnahme der Förderleistung und des Förderdrucks.

Zur erfindungsgemäß verringerbaren Schädigung des Schlauchs ist die sogenannte Spallation von Schlauchmaterialpartikeln zu nennen, die ebenfalls vorteilhaft verringert werden kann. Solche abgesprengten oder abgesplitterten Schlauchpartikel können das gepumpte Fluid verunreinigen. Im medizinischen Bereich kann es beispielsweise zu ihrer Einlagerung in Organen führen.

Die erfindungsgemäße Fördervorrichtung kann vorteilhaft die Querbewegung des Schlauchs (also in einer Richtung parallel zur Drehachse des drehenden Rotors) im Stator reduzieren oder gar vermeiden. Dies wiederum reduziert oder vermindert mittelbar den Abrieb am Schlauch aufgrund der Gleitreibung, erhöht damit die Lebensdauer des Schlauchs, und vermindert das Phänomen der Spallation. Dies betrifft insbesondere den okkludierten Schlauchabschnitt auf der Unterdruckseite.

Von Vorteil kann zudem sein, dass bei Verwenden der erfindungsgemäßen Fördervorrichtung durch eine solche günstige Konstruktion derselben, nicht nur die Lebensdauer des Schlauchs wesentlich erhöht, sondern zudem konstantere Durchflusswerte gehalten werden können.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren der Zeichnung gilt:
- **Fig. 1**: zeigt eine schematische Darstellung einer herkömmlichen Fördervorrichtung;
- **Fig. 2a**: zeigt eine schematische Darstellung einer ersten Ausführungsform der erfindungsgemäßen Fördervorrichtung in einer Ansicht von vorne;
- **Fig. 2b**: zeigt die schematische Darstellung der erfindungsgemäßen Fördervorrichtung der Fig. 2a bei eingelegtem Schlauch;
- **Fig.** 3: zeigt eine schematische Darstellung der erfindungsgemäßen Fördervorrichtung der Fig. 2a mit Blick von unten auf eine Okklusionsvorrichtung und ein axiales Führungselement;
- **Fig.4**: zeigt eine schematische Darstellung des Rotors der Fig. 2a in der Ansicht gemäß Fig. 3 während des Einlegens eines Schlauchs mithilfe der Führungslasche;
- **Fig. 5**: zeigt eine schematische Darstellung des Rotors der vorangegangenen Figuren in der Ansicht der Fig. 4 bei eingelegtem Schlauch;
- **Fig. 6**: zeigt eine schematisch vereinfachte Schnittdarstellung des Rotors der vorangegangenen Figuren bei eingelegtem Schlauch;
- **Fig. 7**: zeigt den Rotor der vorangegangenen Figuren in Perspektive mit Blick von schräg unten und vorne auf dessen Schlauchführungsbereich;
- **Fig. 8**: zeigt eine weitere erfindungsgemäße Ausführungsform der Fördervorrichtung mit Blick auf den Rotor in Perspektive von schräg außen, mit Blick auf eine Okklusionsvorrichtung, ein axiales Führungselement in einer Ausführungsform mit nur einem Seitenabschnitt, sowie mit einem lateralen Führungselement;
- **Fig. 9**: zeigt den Rotor der Fig. 8 in einer Ansicht von vorne;
- **Fig. 10**: zeigt einen Abschnitt einer weiteren erfindungsgemäßen Ausführungsform der Fördervorrichtung mit Blick auf den Rotor in leichter Perspektive von unten;
- **Fig. 11**: zeigt eine schematische Darstellung einer weiteren Ausführungsform der erfindungsgemäßen Fördervorrichtung in Ansicht von vorne ohne Abdeckelement mit Blick auf die Schwingen; und
- **Fig. 12**: zeigt eine schematische Darstellung einer weiteren Ausführungsform der erfindungsgemäßen Fördervorrichtung in Ansicht von vorne ohne Abdeckelement mit Blick auf die gemeinsame Achse von Schwinge und axialem Führungselement.

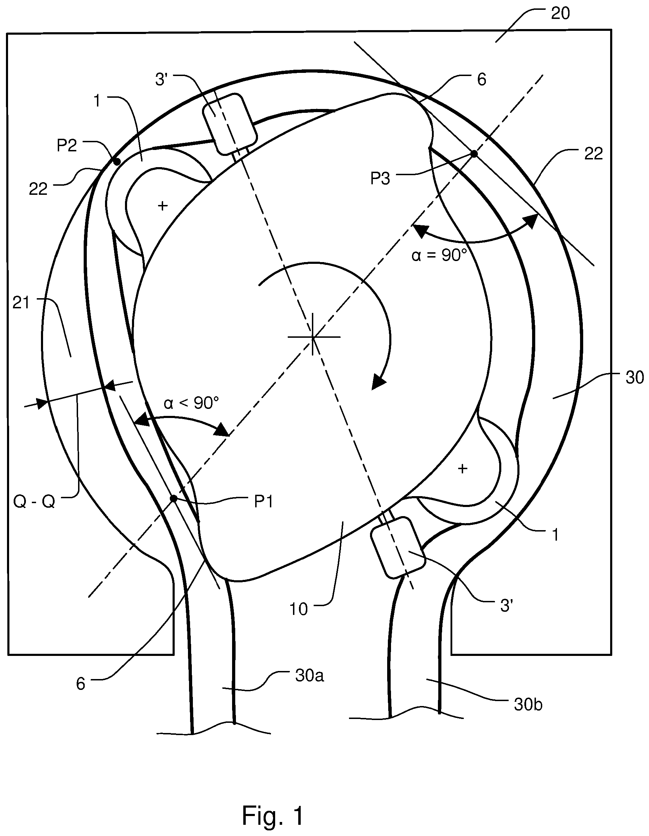

**Fig. 1** zeigt eine schematische Darstellung einer herkömmlichen Fördervorrichtung als Pumpeneinheit mit einem Stator 20 und einem Rotor 10. Der Schlauch 30 befindet sich über dem Statorboden 21 im Raum zwischen Rotor 10 und Stator 20. Der Rotor 10 der Fig. 1 umfasst keine erfindungsgemäß vorgesehenen axialen Führungselemente.

Bei laufendem Rotor 10 finden im Schlauch 30 Querbewegungen (siehe die gegenüberliegenden Pfeile Q-Q) relativ zu den Okklusionsrollen 1 oder zum Führungselement 6 statt. Die Reibungskräfte, die während der Rotation des Rotors auftreten, wirken so auf den Schlauch 30, dass dieser sehnenförmig zwischen Schlaucheingang und der Okklusionsrolle 1 gespannt wird (siehe den Schlauchverlauf von Punkt P1 zu Punkt P2).

Insbesondere in Ausführungsformen der Fördervorrichtung, bei denen der Schlauch 30 am Ein- und Ausgang des Pumpengehäuses (jeweils 30a und 30b) nicht rein tangential, sondern leicht geknickt verläuft, entstehen unter der erwähnten Spannung solche Querbewegungen. Zyklisch wird hier der Schlauch 30 bei jeder kompletten Umdrehung des Rotors 10 gespannt, wobei die Zugspannung bei weiterem Umlaufen der Okklusionsvorrichtung 1 in Richtung zur Überdruckseite wieder abnimmt. Auf der Überdruckseite finden Querbewegungen kaum mehr statt, da der Schlauch 30 dort nur gestaucht wird und sich an das Schlauchbett anlegt.

Bei diesem sehnenförmig gespannten Schlauchabschnitt ist der Winkel zwischen der Achse der Führungsfläche und der Schlauchachse kleiner als 90°, was neben dem Abrollen auch zum Gleiten des Schlauchs über die Rolle führt. Dieser Effekt begünstigt den Abrieb am Schlauch 30 zusätzlich zur beschriebenen Querbewegung.

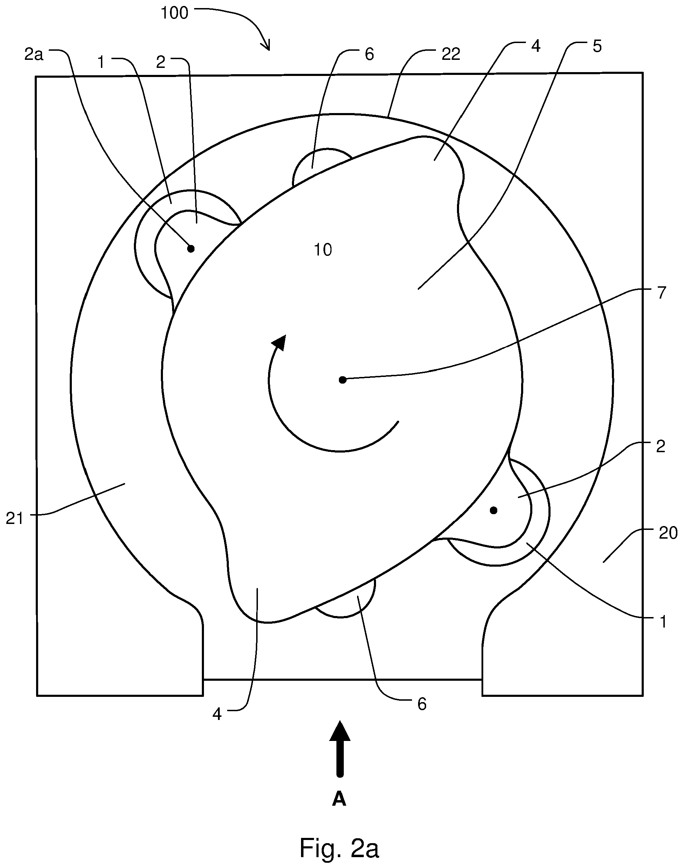

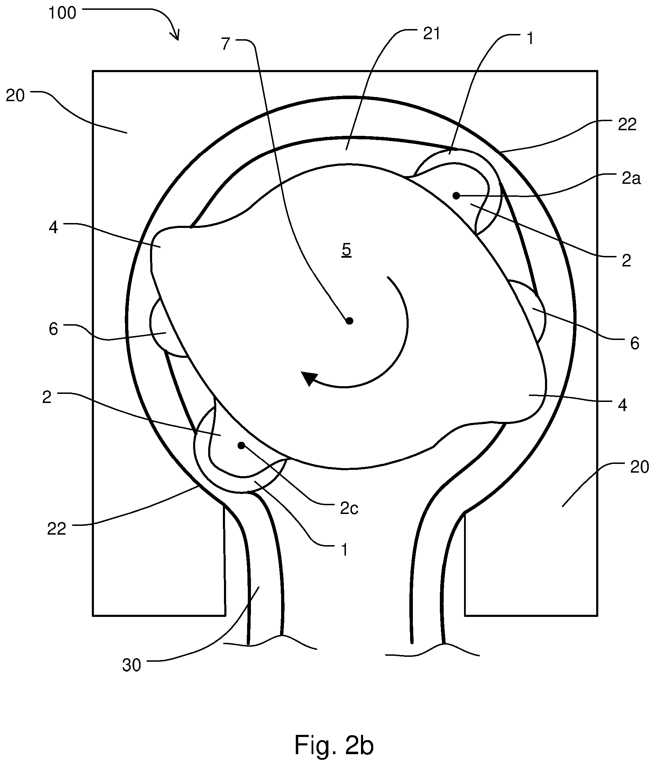

**Fig. 2a** und **Fig. 2b** zeigen jeweils eine schematische Darstellung einer Ausführungsform einer erfindungsgemäßen Fördervorrichtung 100 in einer Ansicht von vorne oder von außen (wie vom Benutzer im Gebrauch wahrgenommen) mit einem um eine Rotorachse 7 rotierenden Rotor 10 und einem feststehenden Stator 20, in welchem sich der Rotor 10 drehend bewegen kann. Der Stator 20 bildet eine Aufnahme für den Rotor 10 und für einen Schlauch 30 (siehe Fig. 2), welcher von unten in den Rotor 10 eingelegt wird und nach unten aus diesem wieder austritt.

Die Aufnahme für den Rotor 10, die in Fig. 2a und 2b hinter dem Rotor 10 liegt, ist entweder als Teil eines Gehäuses (nicht abgebildet) einer Fluidbehandlungsvorrichtung oder als Kassette ausgeführt, welche auf ein Rotorgetriebe der nicht dargestellten Fluidbehandlungsvorrichtung aufgesteckt wird.

Der Rotor 10 kann über eine Welle mit dem Antriebmotor der nicht dargestellten Fluidbehandlungsvorrichtung verbunden werden und von ihm angetrieben werden.

In bestimmten Ausführungsformen kann die Aufnahme für den Rotor 10 eine Antriebswelle aufweisen oder eine solche sein, und auf sie wird der Rotor 10 gesteckt.

Ein Aufnahmeabschnitt des Stators 20 oder Raum für den Schlauch 30 wird gebildet durch einen Statorboden 21, von welchem sich ein Schlauchbett 22 als Gegenlager für die Okklusionsvorrichtung 1 erstreckt. Der Schlauch 30 wird derart in die Fördervorrichtung 100 eingelegt, dass er zwischen Rotor 10 und Schlauchbett 22 als Gegenlager liegt und zumindest abschnittsweise um den Rotor 10 herumgeführt wird, wie in Fig. 2b gezeigt.

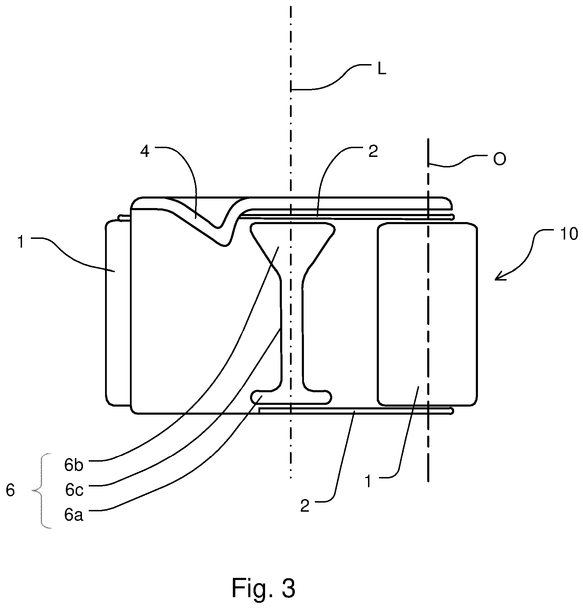

Der Rotor 10 umfasst eine oder mehrere Okklusionsvorrichtung(en) 1, welche beispielsweise als im Wesentlichen zylindrische Okklusionsrollen ausgestaltet sind und welche vorzugsweise um ihre Mitteldrehachse O (siehe Fig. 3) rotieren können. In Fig. 2a sind beispielhaft zwei Okklusionsvorrichtungen 1 zu sehen, welche sich gegenüberliegen und an einer Schwinge 2 angebracht sind. Die Schwinge 2 verbindet die Rotorachse 7 mit den Okklusionsvorrichtungen 1 und koppelt diese funktionell an die rotierende Bewegung des Rotors 10 an. So wird der Schlauch 30 im Gebrauch der Fördervorrichtung 100 durch eine oder mehrere Okklusionsvorrichtungen 1 zyklisch gegen das Schlauchbett 22 als Gegenlager des Stators 20 gepresst und damit okkludiert.

Wenn sich der Rotor 10 dreht, gleiten oder rollen die Okklusionsvorrichtungen 1 entlang des Schlauchs 30 (in Fig. 2a nicht gezeigt). Nachdem die Okklusionsvorrichtungen 1 wieder außer Eingriff mit dem Schlauch 30 gelangt sind, entfaltet sich der zuvor komprimierte Abschnitt des Schlauchs aufgrund der Elastizität des Schlauchmaterials wieder. Der erzeugte Unterdruck saugt das sich stromauf der Fördervorrichtung 100 im Schlauchsystem befindende Fluid an. Der Unterdruck wird durch die Art der Förderung des eingeschlossenen und vor der Okklusionsvorrichtung 1 befindlichen Volumens erzeugt. Auch hierdurch wird das Fluid entlang des Lumens des Schlauchs 30 gefördert.

Dabei können die Okklusionsvorrichtungen 1 den Schlauch 30 mehr oder weniger stark komprimieren. Der Grad dieser Kompression des Schlauchs 30 kann erfindungsgemäß optional eingestellt werden. Beispielsweise kann er mittels einer optionalen, vorzugweise gefederten Schwinge 2 festgesetzt werden.

Mittels einer solchen optionalen Schwinge 2 kann die Fördervorrichtung 100 an die Eigenschaften des konkret verwendeten Schlauchs 30 angepasst werden, z. B. an dessen Materialeigenschaften oder dessen Querschnitt.

Weiter umfasst die Fördervorrichtung 100 der Fig. 2a und Fig. 2b weiter wenigstens ein axiales Führungselement 6 (siehe Fig. 3), hier exemplarisch zwei axiale Führungselemente 6, von denen jeweils ein erster Seitenabschnitt zu erkennen ist, siehe Fig. 3.

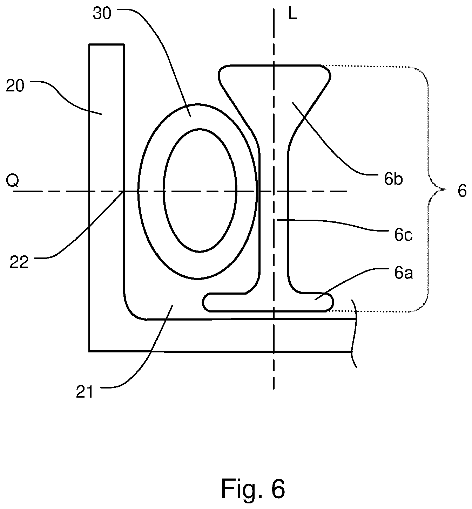

Die axialen Führungselemente 6 begünstigen eine Einbettung des Schlauchs 30, indem sie ihn seitlich (siehe Fig. 3) und von unten abstützen und abgrenzen können, wie z. B. in Fig. 6 detaillierter gezeigt.

Der Rotor 10 der Fig. 2a und Fig. 2b liegt optional unter einem Abdeckelement 5, welches den Benutzer davor schützen kann, Rotorbestandteile versehentlich zu berühren. Das Abdeckelement dient optional auch als Griff zum manuellen Einfädeln des Schlauchs 30.

Das Abdeckelement 5 weist optional zwei Führungslaschen 4 auf oder besteht aus ihnen. Die Führungslaschen 4 können ausgestaltet sein, um mittels ihrer Geometrie das Einfädeln des Schlauchs 30 zu erleichtern (siehe Fig. 3).

Der mit dem Bezugszeichen A bezeichnete Pfeil illustriert die Blickrichtung gemäß der Fig. 3 bis Fig. 5.

**Fig. 3** zeigt eine schematische Darstellung nur des Rotors 10 der Fig. 2a und Fig. 2b mit Blick von unten in Richtung des Pfeils A der Fig. 2a.

Die Okklusionsvorrichtung 1 und das axiale Führungselement 6 sind hier gut zu erkennen, da noch kein Schlauch 30 eingelegt wurde.

Die axialen Führungselemente 6 können unterschiedliche geometrische Konfigurationen aufweisen. Sie können aus einem oder aus mehreren Teilen hergestellt sein. Sie umfassen wenigstens einen mittleren Abschnitt 6c und wenigstens einen ersten, radial über den mittleren Abschnitt 6c überstehenden Seitenabschnitt 6a. Dieser erste Seitenabschnitt 6a steht auch über einen Abschnitt des Außenrands des Rotors 10 radial vor. Die axialen Führungselemente 6 sind vorgesehen, um den Schlauch 30 zu zentrieren oder auszurichten. Sie sind derart konfiguriert, dass Quergleitbewegungen oder Scherbewegungen des Schlauchs 30 bei laufendem Rotor 10 verhindert oder minimiert werden. Um die Reibkräfte, welche auf den Schlauch 30 ausgeübt werden, weiter zu minimieren, kann das axiale Führungselement 6, wie in dieser Ausführungsform, aus sich passiv drehenden Teilen, oder Rollen, bestehen oder solche aufweisen.

Vom axialen Führungselement 6 ist hier nur der erste Seitenabschnitt 6a zu sehen, der mit dem mittleren Abschnitt 6c verbunden ist, siehe Fig. 3. Ein optionaler, zweiter Seitenabschnitt 6b kann vorgesehen sein, siehe z. B. Fig. 3. Er kann mittels des mittleren Abschnitts 6c mit dem ersten Seitenabschnitt 6a verbunden sein.

Erfindungsgemäß können somit beispielsweise der mittlere Abschnitt 6c und/oder der erste Seitenabschnitt 6a und/oder der zweite Seitenabschnitt 6b jeweils um ihre Längsachse (oder um einen Mittelpunkt im Fall einer Ausführungsform mit einem scheibenförmigen, ersten Seitenabschnitt 6a) drehbar sein.

Sowohl die Okklusionsvorrichtung 1 als auch das axiale Führungselement 6 sind hier exemplarisch an einer gemeinsamen Schwinge 2 angeordnet.

Die Okklusionsvorrichtung 1 ist optional zylindrisch ausgestaltet.

Das axiale Führungselement 6 der vorliegenden Ausführungsform ist einteilig, hat einen zylindrischen mittleren Abschnitt 6c, einen ersten Seitenabschnitt 6a, welcher im Wesentlichen scheibenförmig ist, sowie einen zweiten Seitenabschnitt 6b, welcher im Wesentlichen kegelförmig ist.

Der mittlere Abschnitt 6c weist hier ferner beispielhaft einen geringeren maximalen Durchmesser als die Seitenabschnitte 6a und 6b auf. Das axiale Führungselement 6 der Fig. 3 rotiert im Gebrauch um seine zentrale Längsachse L.

In einigen Ausführungsformen rotiert das axiale Führungselement 6 passiv, d. h. seine Achse oder Welle ist nicht mit der Rotorachse mechanisch verbunden. Eine Rotationsbewegung des axialen Führungselements 6 um seine Längsachse wird lediglich durch die Reibung gegen den Schlauch 30 bestimmt, ohne dass weitere Kräfte für diese Rotationsbewegung des axialen Führungselements 6 eine Rolle spielen würden.

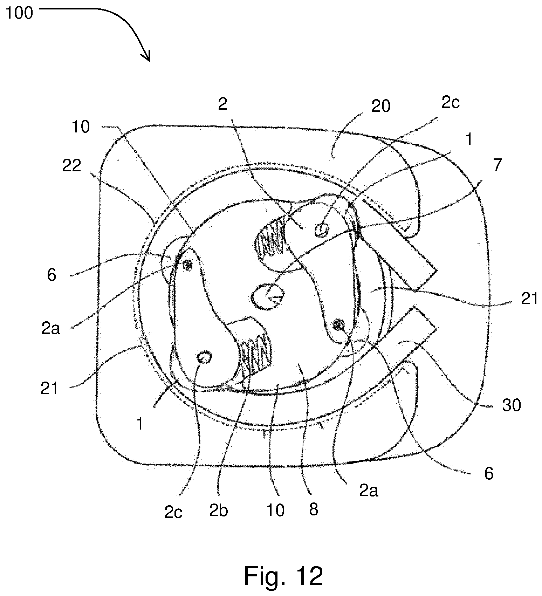

Jedes der axialen Führungselemente 6 kann jeweils einzeln an einer konkreten Schwinge 2 angebracht sein. Die axialen Führungselemente 6 können alternativ paar- oder gruppenweise an einer jeweils gemeinsamen Schwinge 2 angebracht sein. Alternativ oder ergänzend kann wenigstens eines der axialen Führungselemente 6 an einer gemeinsamen Schwinge 2 mit wenigstens einer der Okklusionsvorrichtungen 1 angeordnet sein (siehe z. B. die beispielhafte, erfindungsgemäße Ausführungsform der Fig. 12.

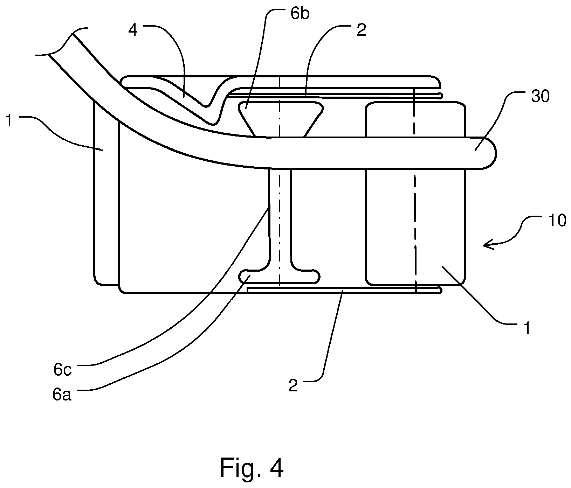

**Fig. 4** zeigt eine schematische Darstellung des Rotors 10 während des Einlegens eines Schlauchs 30 in die Fördervorrichtung 100 unter Zuhilfenahme der Führungslasche 4.

Dabei ist die Führungslasche 4 (allgemein: Führungselement) ausgestaltet, um mittels des axialen Führungselements 6 den Schlauch 30 einfach und mit nur geringer mechanischer Belastung für diesen zwischen Rotor 10 und Stator 20 einzuführen.

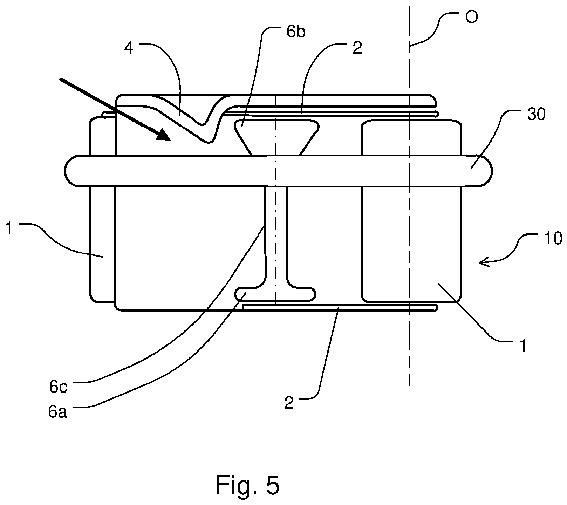

**Fig. 5** zeigt die schematische Darstellung des Rotors 10 der Fig. 4 bei bereits eingelegtem Schlauch 30.

Die geometrische Ausgestaltung des Führungselements 6 ist hier beispielhaft derart, dass der Schlauch 30 bei laufendem Rotor 10 nicht mit der Führungslasche 4 in Berührung kommt. Dies ist in Fig. 5 durch einen dünnen Pfeil illustriert.

Aus Fig. 5 ist ersichtlich, wie der Schlauch über den mittleren Abschnitt 6c und in Kontakt mit diesem geführt und dabei mittels des ersten Seitenabschnitts 6a und des zweiten Seitenabschnitts 6b des axialen Führungselements 6 jeweils lateral begrenzt wird.

Die Geometrie des axialen Führungselements 6 hält den Schlauch 30 zentriert um den Rotor 10 im Statorboden 21 derart, dass der Schlauch 30 bei laufendem Rotor 10 weder mit dem Statorboden 21, der Führungslasche 4 und/oder dem Abdeckelement 5 in Berührung kommt.

Auf diese Weise hat der zwischen Okklusionsvorrichtungen 1 und Stator 20 eingelegte Schlauch 30 vorteilhafterweise nur ein geringes seitliches oder axiales Spiel, d. h. der Schlauch 30 ist kaum Querbewegungen bei laufendem Rotor und kaum Scherbewegungen ausgesetzt. Die Minderung des Schlauchabriebs kann sich materialschonend auswirken.

**Fig. 6** zeigt eine stark vereinfachte Schnittdarstellung eines Abschnittes des Stators 20 mit einem zwischen Stator 20 und Rotor 10 eingefügten Abschnitt des Schlauchs 30 und einem axialen Führungselement 6 im Detail.

Durch geeignete Auswahl der Konstruktionsparameter des axialen Führungselements 6 kann die Lebensdauer des Schlauchs 30 wesentlich erhöht werden. In einigen Ausführungsformen können ferner konstantere Durchflusswerte entlang des Schlauchs 30 gehalten werden.

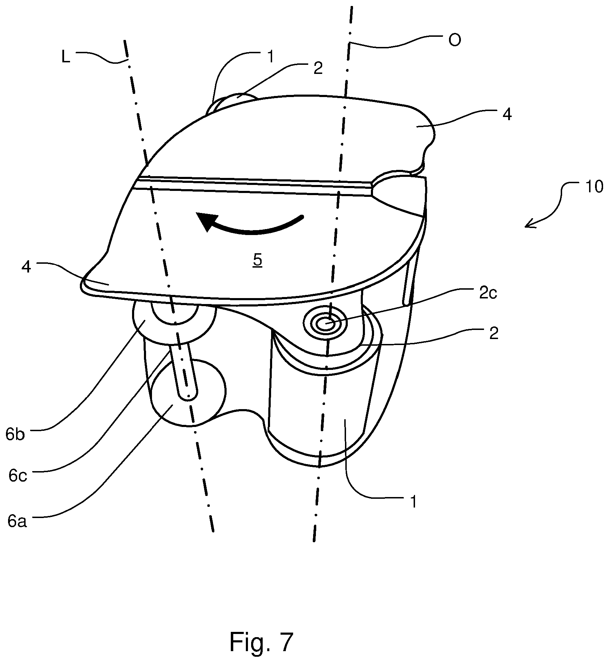

**Fig. 7** zeigt eine schematische Darstellung des Rotors 10 der vorangegangenen Figuren in Perspektive mit Blick von schräg unten und vorne (außen) auf den Bereich, in dem der Schlauch 30 um den Rotor 10 geführt werden kann.

Der Rotor 10 umfasst hier das optionale Abdeckelement 5, das den Rotor 10 im Wesentlichen bedeckt. In radialer Richtung stehen nur die Schwingen 2 mit den Okklusionsvorrichtungen 1 und den axialen Führungselementen 6 über das Abdeckelement 5 des Rotors 10 über. Gut zu erkennen ist die Achse 2c der Okklusionsvorrichtung 1, ausgestaltet als Schwingachse, mit welcher diese in bzw. an der Schwinge 2 gelagert ist. Diese Achse 2c ermöglicht die Rotation der Okklusionsvorrichtung 1 um ihre zentrale Längsachse O.

In dieser Ausführungsform sind die beiden Seitenabschnitte 6a und 6b des axialen Führungselements 6 beispielhaft scheibenförmig ausgestaltet. Sie weisen optional einen gleich großen Durchmesser auf. Der mittlere Abschnitt 6c ist zylinderförmig ausgestaltet und weist einen wesentlich geringeren Durchmesser auf als die beiden Seitenabschnitte 6a, 6b. Beide Seitenabschnitte 6a, 6b können optional kegelförmig sein, wobei die jeweilige Kegelhöhe der Seitenabschnitte 6a, 6b in manchen Ausführungsformen gleich, in anderen Ausführungsformen unterschiedlich sein kann.

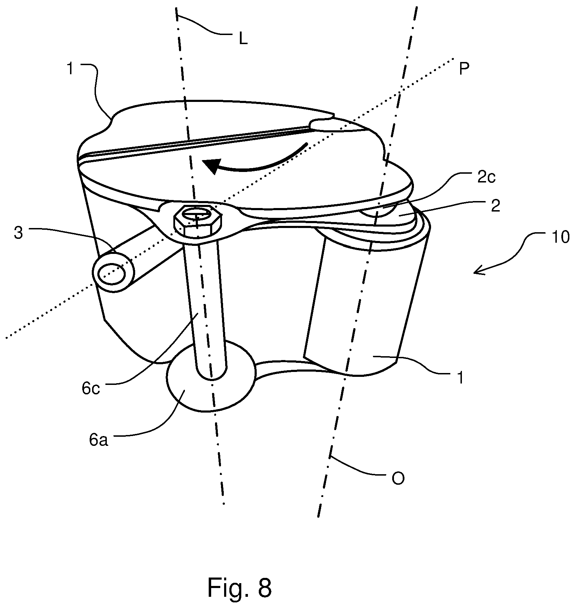

**Fig. 8** zeigt eine Darstellung einer weiteren Ausführungsform der Fördervorrichtung mit Blick auf den Rotor 10 in Perspektive mit Blick von schräg unten und vorne. Die Okklusionsvorrichtung 1, ein axiales Führungselement 6 sowie ein laterales Führungselement 3 lassen sich gut erkennen.

In manchen Ausführungsformen, z. B. in der der Fig. 8, weist das axiale Führungselement 6 nur den ersten Seitenabschnitt 6a und den mittleren Abschnitt 6c auf, insbesondere aber nicht den zweiten Seitenabschnitt 6b.

Der erste Seitenabschnitt 6a grenzt den Schlauch 30 zum Statorboden 21 hin ab. Er hat eine geometrische Ausgestaltung, beispielsweise (vorzugsweise vollständig oder im Wesentlichen) scheiben- oder kegelförmig, die geeignet ist, den Schlauch 30 bei laufendem Rotor 10 derart zentriert zu halten, dass keine unbeabsichtigte Berührung des Statorbodens 21 oder von Teilen bzw. Bereichen des Rotorkörpers (beispielsweise des Nabenkörpers 8) erfolgen kann.

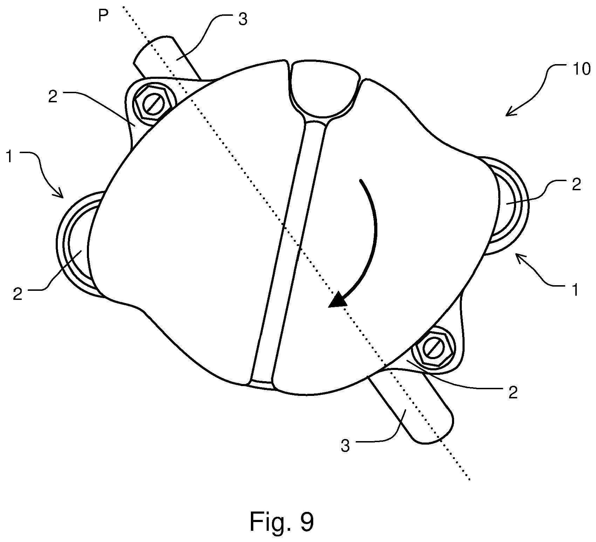

Weiter weist der Rotor 10 in dieser Ausführungsform wenigstens ein laterales Führungselement 3 auf (siehe auch Fig. 9). Die lateralen Führungselemente 3, von denen in Fig. 8 nur eines zu sehen ist, grenzen den Schlauch 30 lateral zur Außenseite des Rotors 10, das heißt zur offenen Seite des Raums zwischen Rotor 10 und Stator 20, also vom Statorboden 21 weg oder zum Benutzer hin, ab. Somit verhindern sie, dass der Schlauch 30 bei laufendem Rotor 10 aus der Fördervorrichtung 100 herausgleitet. Im Zusammenwirken mit dem axialen Führungselement 6 verhindern die lateralen Führungselemente 3, dass Quergleit- oder Scherbewegungen des Schlauchs 30 entstehen können.

Das wenigstens eine laterale Führungselement 3 kann als zylindrischer oder im Wesentlichen zylindrischer Körper ausgestaltet sein oder einen solchen aufweisen, welcher durch eine seiner Grundflächen an einer Achse angeordnet ist, welche sich radial zur Rotorachse 7 wegstreckt. Die Längsachse des lateralen Führungselements 3 steht vorzugweise senkrecht zur Längsachse der Rotorachse 7. Das laterale Führungselement 3 kann optional um seine Längsachse P rotieren, um die Reibungskräfte gegen den Schlauch 30 bei laufendem Rotor zu reduzieren.

**Fig. 9** zeigt den Rotor 10 der Fig. 8 in einer Ansicht von vorne.

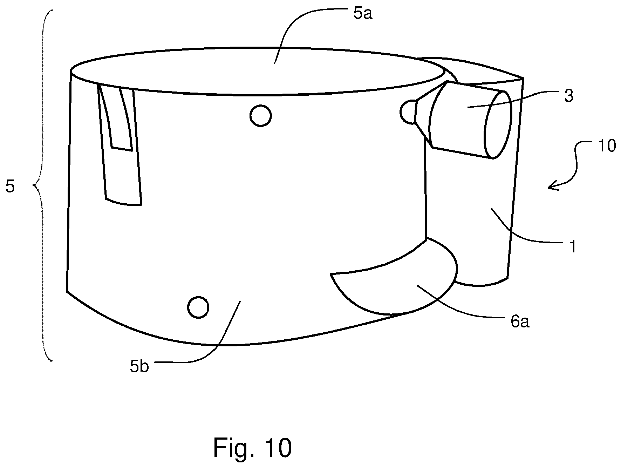

**Fig. 10** zeigt einen Abschnitt einer wiederum weiteren Ausführungsform der Fördervorrichtung 100 mit Blick auf den Rotor 10 in leichter Perspektive von unten.

Hier bildet das Abdeckelement 5 ein Gehäuse mit einer Oberfläche oder Fläche 5a, welche den Rotor 10 nach außen zum Benutzer hin abdeckt und welche die Führungslaschen 4 umfasst, sowie mit einer Mantelfläche oder Umrandung 5b, welche sich optional bis zum Statorboden 21 hin oder nahe an diesen heran erstreckt, und welche Aussparungen aufweist, durch welche die Okklusionsvorrichtungen 1, die Achse des wenigstens einen lateralen Führungselements 3 und/oder Seitenabschnitte des wenigstens einen axialen Führungselements 6, oder Abschnitte hiervon, aus dem Rotor 10 hervortreten können.

Beispielsweise ist in dieser Ausführungsform der mittlere Abschnitt 6c des axialen Führungselements 6 im Gehäuse integriert. Der hier scheibenförmig ausgestaltete erste Seitenabschnitt 6a des axialen Führungselements 6 ist hierbei exemplarisch als rotierendes Teil konfiguriert und kann deutlich seitlich aus dem Abdeckelement 5 hervortreten.

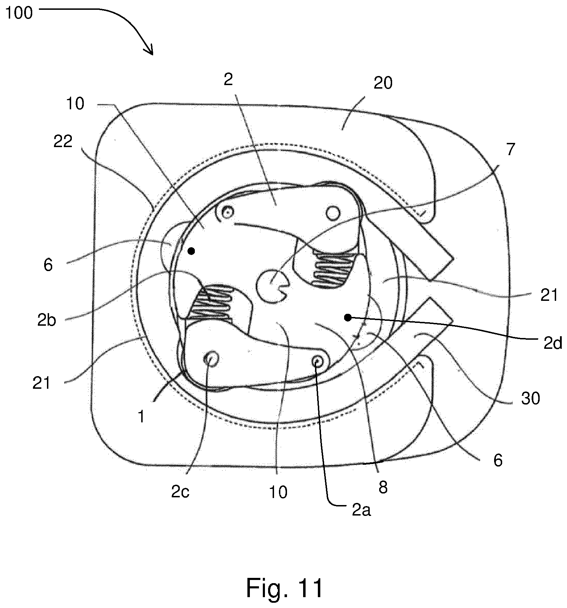

**Fig. 11** zeigt eine schematische Darstellung einer weiteren Ausführungsform der Fördervorrichtung 100 in leichter Perspektive von vorne mit Blick auf die Schwinge(n) 2.

Die Schwinge 2 kann alle aus dem Stand der Technik bekannten Ausgestaltungen haben. Sie kann beispielsweise als Gabel mit einem Schwinggelenk 2a ausgestaltet sein, welches die Gabel mit einem Nabenkörper 8 verbindet. Außerdem kann sie mit einer Schwingfeder 2b ausgestaltet sein, sowie mit einer Achse 2c, um die Okklusionsvorrichtung 1 an der Gabel der Schwinge 2 frei rotierend anzubringen.

Der Nabenkörper 8 koppelt die Schwinge 2 und damit alle an ihr angeordneten Teile am Rotor 10 an, welcher über eine Welle eines nicht dargestellten Antriebsmotors der nicht dargestellten Blutbehandlungsvorrichtung angetrieben wird. Der Nabenkörper 8 koppelt in diesen Ausführungsformen beispielsweise alle Komponenten des Rotors 10, die sich im Rotor 10 radial erstrecken, insbesondere Okklusionsvorrichtungen 1 und axiale Führungselemente 6 mittels einer Achse 2d, an die Rotation desselben.

In der dargestellten Ausführungsform der Fig. 11 sind die axialen Führungselemente 6 nicht an einer der Schwingen 2 angeordnet.

Die Schwinge(n) 2 können rigide Strukturen oder leicht federnd (wie in den Fig. 11 und 12 dargestellt) sein. Eine leichte Federung der Schwinge(n) kann beispielsweise vorteilhaft dazu beitragen, die Anpassung des Rotors 10 an unterschiedliche Schläuche, z. B. Schläuche mit unterschiedlichen Durchmessern, zu ermöglichen.

Grundsätzlich sind auch unterschiedliche Ausgestaltungen der Schwingen 2 in einer einzelnen Ausführungsform des Rotors 10 von der vorliegenden Erfindung mit umfasst.

**Fig. 12** zeigt eine schematische Darstellung einer weiteren Ausführungsform der erfindungsgemäßen Fördervorrichtung 100 mit einer Schwinge 2 und ohne Abdeckelement mit Blick von vorne.

Der Aufbau der hier vorliegenden Ausführungsform entspricht ebenfalls größtenteils dem Aufbau der Ausführungsform der Fig. 11. Jedoch ist hier jeweils wenigstens seine Okklusionsvorrichtung 1 mittels einer Schwinge 2 mit einem axialen Führungselement 6 verbunden, wobei das Schwinggelenk 2a als Achse für das axiale Führungselement 6 dient. Damit liegen wenigstens eine Okklusionsvorrichtung 1 und wenigstens ein axiales Führungselement 6 an einer gemeinsamen Schwinge 2 vor.

Die Okklusionsvorrichtungen 1 können ferner mittels der Schwingfedern 2b federnd gelagert sein, was einem leichteren Einbringen eines Schlauchs 30 in den Rotor 10 dienen und/oder die Anpassung des Rotors 10 an unterschiedliche Schläuche, wie hierin beschrieben, erleichtern kann.

Die oben zur Fig. 11 gemachten Ausführungen, insbesondere zum Nabenkörper 8, treffen ungemindert auch auf die Ausführungen oder Darstellungen der Fig. 12 zu.

### Bezugszeichen

- 1: Okklusionsvorrichtung
- 2: Schwinge
- 2a: Schwinggelenk
- 2b: Schwingfedern
- 2c: Achse der Okklusionsvorrichtung
- 2d: Achse des Führungselements
- 3: laterales Führungselement
- 4: Führungslasche
- 5: Abdeckelement
- 5a: Fläche, Oberfläche
- 5b: Umrandung, Mantelfläche
- 6: axiales Führungselement
- 6a: erster Seitenabschnitt zur Maschinenseite des axialen Führungselements
- 6b: zweiter Seitenabschnitt zur Benutzerseite des axialen Führungselements
- 6c: mittlerer Abschnitt des axialen Führungselements
- 7: Antriebswelle, oder Rotorwelle oder Achse
- 8: Nabenkörper
- 10: Rotor
- 20: Stator
- 21: Statorboden
- 22: Schlauchbett als Gegenlager für die Okklusionsvorrichtungen
- 30: Schlauch
- 100: Fördervorrichtung
- L: zentrale Längsachse des axialen Führungselements
- Q: Mittelquerachse des axialen Führungselements
- O: zentrale Längsachse der Okklusionsvorrichtung
- P: zentrale Längsachse des lateralen Führungselements

## Patentansprüche

1. Fördervorrichtung (100) zum Fördern eines in einem Schlauch (30) geführten medizinischen Fluids mit einem Rotor (10) und einem Stator (20), wobei der Stator (20) mindestens einen Statorboden (21), einen Raum für die Aufnahme des Schlauchs (30) in der Fördervorrichtung (100) und ein Schlauchbett (22) als Gegenlager für Okklusionsvorrichtungen (1) aufweist, wobei der Rotor (10) wenigstens eine Rotorachse (7) und wenigstens zwei Okklusionsvorrichtungen (1) aufweist, welche radial zur Rotorachse (7) angebracht sind und im Gebrauch der Fördervorrichtung (100) den Schlauch (30) intermittierend gegen das Schlauchbett (22) komprimieren, wobei der Rotor (10) wenigstens ein um seine Längsachse drehbar angeordnetes axiales Führungselement (6) zum Ausrichten oder zum Zentrieren des Schlauchs (30) im Stator (20) umfasst, wobei die Längsachse des axialen Führungselements (6) parallel zur Längsachse der Rotorachse (7) verläuft, und wobei das axiale Führungselement (6)
- wenigstens einen mittleren Abschnitt (6c) und wenigstens einen ersten, radial über den mittleren Abschnitt (6c) überstehenden Seitenabschnitt (6a) umfasst, wobei wenigstens der erste Seitenabschnitt (6a) rotierbar angeordnet ist; und wobei
- der erste Seitenabschnitt (6a) abschnittsweise radial über einen Abschnitt eines Außenrands des Rotors (10) übersteht.

2. Fördervorrichtung (100) nach Anspruch 1, mit wenigstens einem weiteren axialen Führungselement (6), wobei wenigstens eines der axialen Führungselemente (6) oder wenigstens eine der Okklusionsvorrichtungen (1) an einer Schwinge (2) des Rotors (10) angeordnet sind.

3. Fördervorrichtung (100) nach Anspruch 1 oder 2, wobei wenigstens zwei axiale Führungselemente (6) und/oder wenigstens zwei Okklusionsvorrichtungen (1) einander gegenüberliegend an einer gemeinsamen Schwinge (2) des Rotors (10) angeordnet sind.

4. Fördervorrichtung (100) nach einem der vorangegangenen Ansprüche, wobei mehrere Okklusionsvorrichtungen (1) und mehrere axialen Führungselemente (6) des Rotors (10) an einer Schwinge (2) derart angeordnet sind, dass die axialen Führungselemente (6) und die Okklusionsvorrichtungen (1) im Rotor (10) abwechselnd angeordnet sind.

5. Fördervorrichtung (100) nach einem der vorangegangenen Ansprüche, wobei wenigstens eines der axialen Führungselemente (6) wenigstens einen zweiten Seitenabschnitt (6b) aufweist, welcher kegelförmig ist.

6. Fördervorrichtung (100) nach einem der vorangegangenen Ansprüche, wobei der erste und der zweite Seitenabschnitt (6a, 6b) des axialen Führungselements (6) kegelförmig sind und zusammen einen Doppelkegel bilden, wobei der mittlere Abschnitt (6c) des axialen Führungselements (6) zwischen dem ersten und dem zweiten Seitenabschnitt (6a, 6b) angeordnet ist und diese miteinander verbindet.

7. Fördervorrichtung (100) nach Anspruch 6, wobei die kegelförmigen, ersten und zweiten Seitenabschnitte (6a, 6b) unterschiedlich sind, sodass der Doppelkegel nicht punktsymmetrisch zu seinem geometrischen Schwerpunkt ist.

8. Fördervorrichtung (100) nach einem der vorangegangenen Ansprüche, wobei der Rotor (10) ferner wenigstens ein laterales Führungselement (3) umfasst, dessen Längsachse senkrecht zur Rotorachse (7) verläuft, und welches gegenüber dem Statorboden (21) angeordnet ist und den Schlauch (30) nur nach vorne oder außen begrenzt.

9. Fördervorrichtung (100) nach Anspruch 8, wobei das laterale Führungselement (3) um seine Mittellängsachse (P) drehbar angeordnet ist.

10. Fördervorrichtung (100) nach einem der vorangegangenen Ansprüche, wobei nur der mittlere Abschnitt (6c) des axialen Führungselements (6) um seine Mittellängsachse (L) drehbar ist.

11. Fördervorrichtung (100) nach einem der vorangegangenen Ansprüche mit einem Abdeckelement (5), welches den Rotor (10) abdeckt oder zumindest teilweise bedeckt.

12. Fördervorrichtung (100) nach einem der vorangegangenen Ansprüche, wobei das Abdeckelement (5) wenigstens eine Führungslasche (4) für ein einfaches und sicheres Einbringen des Schlauchs (30) in den Raum zwischen Rotor (10) und Stator (20) aufweist, oder aus einer solchen besteht.

13. Fördervorrichtung (100) nach einem der vorangegangenen Ansprüche, wobei wenigstens ein axiales Führungselement (6) und/oder ein laterales Führungselement (3) in das Abdeckelement (5) integriert sind.

14. Blutbehandlungsvorrichtung (1000) mit einer Fördervorrichtung (100) nach einem der vorangegangenen Ansprüche.

15. Blutbehandlungsvorrichtung nach Anspruch 14, ausgestaltet als Dialysevorrichtung, Hämodialysevorrichtung, Hämofiltrationsvorrichtung, Hämodiafiltrationsvorrichtung, Apheresevorrichtung, Plasmabehandlungs- oder Austauschvorrichtung, TPE-Vorrichtung, und/oder Kombinationen hiervon.

## Claims

1. A conveying device (100) for conveying a medical fluid guided into a tube (30), having a rotor (10) and
a stator (20), wherein the stator (20) comprises at least one stator base (21), a space for receiving the tube (30) in the conveying device (100) and a tube bed (22) as a counter bearing for occlusion devices (1),
wherein the rotor (10) comprises at least one rotor axis (7) and at least two occlusion devices (1) which are mounted radially to the rotor axis (7) and intermittently compress the tube (30) against the tube bed (22) during use of the conveying device (100), wherein the rotor (10) comprises at least one axial guiding element (6), arranged to rotate about its longitudinal axis, for aligning or centering the tube (30) in the stator (20), wherein the longitudinal axis of the axial guiding element (6) runs parallel to the longitudinal axis of the rotor axis (7), and wherein the axial guiding element (6)
- comprises at least one middle section (6c) and at least one first lateral section (6a) protruding radially over the middle section (6c), wherein at least the first lateral section (6a) is rotatably arranged; and wherein
- the first lateral section (6a) partially protrudes radially over a section of an outer edge of the rotor (10).

2. The conveying device (100) according to claim 1, comprising at least one further axial guiding element (6), wherein at least one of the axial guiding elements (6) or at least one of the occlusion devices (1) is arranged on a swing arm (2) of the rotor (10).

3. The conveying device (100) according to claim 1 or 2, wherein at least two axial guiding elements (6) and/or at least two occlusion devices (1) are arranged opposite to each other on a common swing arm (2) of the rotor (10).

4. The conveying device (100) according to any one of the preceding claims, wherein a plurality of occlusion devices (1) and a plurality of axial guiding elements (6) of the rotor (10) are arranged on a swing arm (2) such that the axial guiding elements (6) and the occlusion devices (1) are arranged alternately in the rotor (10).

5. The conveying device (100) according to any one of the preceding claims, wherein at least one of the axial guiding elements (6) comprises at least one second lateral section (6b) which is conical.

6. The conveying device (100) according to any one of the preceding claims, wherein the first and the second lateral sections (6a, 6b) of the axial guiding element (6) are conical and together form a double cone, wherein the middle section (6c) of the axial guiding element (6) is arranged between the first and the second lateral sections (6a, 6b) and connects them to each other.

7. The conveying device (100) according to claim 6, wherein the conical, first and second lateral sections (6a, 6b) are different, so that the double cone is not point-symmetrical to its geometric center of gravity.

8. The conveying device (100) according to any one of the preceding claims, wherein the rotor (10) further comprises at least one lateral guiding element (3), whose longitudinal axis runs perpendicular to the rotor axis (7), and which is arranged opposite the stator base (21) and delimits the tube (30) only to the front or outside.

9. The conveying device (100) according to claim 8, wherein the lateral guiding element (3) is arranged rotatably about its central longitudinal axis (P).

10. The conveying device (100) according to any one of the preceding claims, wherein only the middle section (6c) of the axial guiding element (6) is rotatable about its central longitudinal axis (L).

11. The conveying device (100) according to any one of the preceding claims having a cover element (5) which covers or at least partially covers the rotor (10).

12. The conveying device (100) according to any one of the preceding claims, wherein the cover element (5) comprises, or consists of, at least one guiding lug (4) for easy and safe insertion of the tube (30) in the space between rotor (10) and stator (20).

13. The conveying device (100) according to any one of the preceding claims, wherein at least one axial guiding element (6) and/or one lateral guiding element (3) is/are integrated into the cover element (5).

14. A blood treatment apparatus (1000) comprising a conveying device (100) according to any one of the preceding claims.

15. The blood treatment apparatus according to claim 14, designed as a dialysis apparatus, hemodialysis apparatus, hemofiltration apparatus, hemodiafiltration apparatus, apheresis apparatus, plasma treatment or plasma exchange apparatus, TPE apparatus, and/or as combinations thereof.

## Revendications

1. Un dispositif de transport (100) destiné à l'acheminement d'un fluide médical guidé dans un tuyau (30), comprenant un rotor (10) ainsi qu'un stator (20),
où le stator (20) présente au moins une base de stator (21), un espace pour recevoir le tuyau (30) dans le dispositif de transport (100) ainsi qu'un support de tuyau (22) servant de contre-appui aux dispositifs d'occlusion (1),
où le rotor (10) présente au moins un axe de rotor (7) et au moins deux dispositifs d'occlusion (1) montés radialement par rapport à l'axe de rotor (7) et comprimant par intermittence le tuyau (30) contre le support de tuyau (22) lors de l'utilisation du dispositif de transport (100), où le rotor (10) présente au moins un élément de guidage axial (6) agencé pour tourner autour de son axe longitudinal, afin d'aligner ou de centrer le tuyau (30) dans le stator (20), où l'axe longitudinal de l'élément de guidage axial (6) est parallèle à l'axe longitudinal du rotor (7),
et où l'élément de guidage axial (6)
- comprend au moins une partie médiane (6c) et au moins une première partie latérale (6a) faisant saillie radialement sur la partie médiane (6c),
où au moins la première partie latérale (6a) est agencée de manière rotative; et où
- la première partie latérale (6a) fait partiellement saillie radialement sur une section d'un bord extérieur du rotor (10).

2. Le dispositif de transport (100) selon la première revendication, comprenant au moins un autre élément de guidage axial (6), où au moins l'un des éléments de guidage axiaux (6) ou au moins l'un des dispositifs d'occlusion (1) est agencé sur un bras oscillant (2) du rotor (10).

3. Le dispositif de transport (100) selon la revendication 1 ou 2, où au moins deux éléments de guidage axiaux (6) et/ou au moins deux dispositifs d'occlusion (1) sont agencés l'un en face de l'autre sur un bras oscillant commun (2) du rotor (10).

4. Le dispositif de transport (100) selon l'une quelconque des revendications précédentes, où plusieurs dispositifs d'occlusion (1) et plusieurs éléments de guidage axiaux (6) du rotor (10) sont agencés sur un bras oscillant (2) de telle sorte que les éléments de guidage axiaux (6) ainsi que les dispositifs d'occlusion (1) sont agencés de façon alternative dans le rotor (10).

5. Le dispositif de transport (100) selon l'une quelconque des revendications précédentes, où au moins l'un des éléments de guidage axiaux (6) présente au moins une seconde partie latérale (6b) qui est conique.

6. Le dispositif de transport (100) selon l'une quelconque des revendications précédentes, où les première et seconde parties latérales (6a, 6b) de l'élément de guidage axial (6) sont coniques et forment ensemble un double cône, la partie médiane (6c) de l'élément de guidage axial (6) étant agencée entre les première et seconde parties latérales (6a, 6b) et les reliant entre elles.

7. Le dispositif de transport (100) selon la revendication 6, où les première et seconde parties latérales coniques (6a, 6b) sont différentes, de sorte que le double cône n'est pas à symétrie ponctuelle par rapport à son centre de gravité géométrique.

8. Le dispositif de transport (100) selon l'une quelconque des revendications précédentes, où le rotor (10) comprend en outre au moins un élément de guidage latéral (3), dont l'axe longitudinal est perpendiculaire à l'axe du rotor (7), et qui est agencé en face de la base du stator (21) et délimite le tuyau (30) uniquement vers l'avant ou vers l'extérieur.

9. Le dispositif de transport (100) selon la revendication 8, où l'élément de guidage latéral (3) est agencé de manière à pouvoir tourner autour de son axe longitudinal central (P).

10. Le dispositif de transport (100) selon l'une quelconque des revendications précédentes, où seule la partie médiane (6c) de l'élément de guidage axial (6) peut tourner autour de son axe longitudinal central (L).

11. Le dispositif de transport (100) selon l'une quelconque des revendications précédentes, comprenant un élément de recouvrement (5) qui recouvre ou recouvre au moins partiellement le rotor (10).

12. Le dispositif de transport (100) selon l'une quelconque des revendications précédentes, où l'élément de recouvrement (5) comprend, ou est constitué, d'au moins une patte de guidage (4) pour une insertion facile et sûre du tuyau (30) dans l'espace entre le rotor (10) et le stator (20).

13. Le dispositif de transport (100) selon l'une quelconque des revendications précédentes, où au moins un élément de guidage axial (6) et/ou un élément de guidage latéral (3) est/sont intégré(s) dans l'élément de recouvrement (5).

14. Un appareil de traitement du sang (1000) comprenant un dispositif de transport (100) selon l'une quelconque des revendications précédentes.

15. L'appareil de traitement du sang selon la revendication 14, conçu comme un appareil de dialyse, un appareil d' hémodialyse, un appareil d'hémofiltration, un appareil d'hémodiafiltration, un appareil d'aphérèse, un appareil de traitement ou d'échange de plasma, un appareil TPE et/ou des combinaisons de ceux-ci.
